# EUROPEAN PATENT SPECIFICATION

(11) **EP 0 739 185 B1**
(45) Date of publication and mention of the grant of the patent: **29.09.2004**
(21) Application number: 95907412.1
(22) Date of filing: 12.01.1995
(51) Int. Cl.: A61B 17/16, A61F 2/08

(54) **ALLOGENIC OR SYNTHETIC COMPOSITE BONE GRAFT**
ALLOGENES ODER SYNTHETISCHES KNOCHENKOMPOSITIMPLANTAT
GREFFE OSSEUSE COMPOSITE ALLOGENE OU SYNTHETIQUE

(30) Priority: 13.01.1994 US 180956; 30.11.1994 US 347578
(43) Date of publication of application: 30.10.1996
(73) Proprietor: McGuire, David A., Anchorage, AK 99508 (US)
(72) Inventor: McGuire, David A., Anchorage, AK 99508 (US)
(74) Representative: MacGregor, Gordon
(86) International application number: PCT/US1995/000452
(87) International publication number: WO 1995/019141

(56) References cited:
- EP-A- 0 265 659
- WO-A-93/09730
- DE-U- 8 504 736
- FR-A- 2 679 126
- US-A- 4 708 139
- US-A- 4 946 462
- US-A- 5 163 940
- US-A- 5 234 430
- US-A- 5 257 996

## Description

The present invention relates to the field of arthroscopic surgery, particularly anterior cruciate ligament reconstruction.

One common approach to cruciate ligament reconstruction is the use of the patellar tendon to form a bone-tendon-bone graft. This involves cutting out a bone block from the top of the patella. Deviations from proper technique for removal of the patellar bone block have resulted in reports of patellar tendinitis, patellar fractures and even, in some instances, ruptures of the patellar ligament after the grafting procedure. Whether or not these complaints are properly related to the use of the patellar bone block in cruciate ligament reconstruction, these concerns are reason enough to identify an alternate method for performing the surgery.

Cruciate ligament reconstruction involves the drilling of a tunnel through the tibia and into the femur. The tunnels are used for insertion of a graft to replace the damaged cruciate ligament. The accuracy in drilling these tunnels is critical to providing a satisfactory repair. A commonly used method of directing a drill to form these tunnels is by using a guide wire drilled through the bone first. The location of the guide wire can be tested before drilling out the tunnel. A cannulated drill is then inserted over the guide wire to drill the full size tunnel. When it has been desirable to retain the core of the bone tunnel being drilled, a coring reamer is used. However, when a coring reamer is used over a guide wire, the guide wire produces a stress riser in the bone core. Moreover, the use of a guide wire is generally inadequate to accurately guide a coring reamer throughout the drilling of an entire bone tunnel.

### SUMMARY OF THE INVENTION

The present invention is directed to a bone-tendon-bone composite graft as defined in Claim 1 for use in cruciate ligament reconstruction. Further embodiments of the invention are as defined in Claims 2 to 9. The use of the present invention is directed to ligament reconstruction surgery. In a preferred embodiment, a bone tunnel is formed in each of two bones of the joint. In knee surgery, these are the femur and the tibia.

The bone plugs of the invention are machined to form two longitudinal substantially parallel grooves opposite one another. At least one ligament replacement, such as a semitendinosus tendon, and/or gracilis, is extended between both of two bone plugs along the parallel grooves in each plug. The ligament replacement is attached to the two bone plugs. Each bone plug is inserted into one of the bone tunnels and secured therein by an interference screw. The use of the bone-tendon-bone composite graft of the invention results in a reconstructed cruciate ligament.

A bone block drill guide can be used for forming the bone plugs required in the composite graft of the present invention. The bone block drill guide includes a main hole for accommodating the bone plug. First and second parallel holes that intersect opposite sides of the main hole are used for directing a drill bit to cut a groove longitudinally along the bone block.

A trefoil rasp may be used to file channels in the bone tunnels. Two channels, oppositely located from one another, accommodate the ligament replacement attached between the two bone grafts. The third channel is generally located parallel to and equidistant from the other two channels. This third channel is used to guide an interference screw along the bone tunnel adjacent to the bone portion of the graft.

The composite graft in accordance with an embodiment of the present invention is advantageously formed without cutting into the patella. The trefoil rasp provides for interference screw fixation without permitting the screw to cut into the ligament replacement.

Other features of the present invention will become apparent during the following description taken in conjunction with the drawings.

### BRIEF DESCRIPTION OF THE DRAWINGS

FIG. 1 is a perspective view in partial cross section of a reconstructed ligament formed with the present invention.
FIG. 2 is an isometric view of a tibial drill guide which can be used to form the present invention.
FIG. 3a is an isometric view of a trefoil rasp.
FIG. 3b is an end view of the rasp of Fig. 3a.
FIG. 4a is an isometric view of a bone plug made by the invention.
FIG. 4b is a side view of the bone plug of Fig. 4a.
FIG. 5 is a side view of a bone-tendon-bone composite graft made by the present invention.
FIG. 6 is a cross-sectional view of FIG. 1 taken along lines 6-6.
FIG. 7a is a side view of a bone block drill guide.
FIG. 7b is a plan view of the bone block drill guide of FIG. 7a.
FIG. 8 is an isometric view of a tibial guide in use against a knee.
FIG. 9 is an isometric view of the distal end of the positioning arm of the tibial guide of FIG. 8.
FIG. 10 is an isometric view of a coring reamer.

### DETAILED DESCRIPTION OF THE PREFERRED EMBODIMENTS

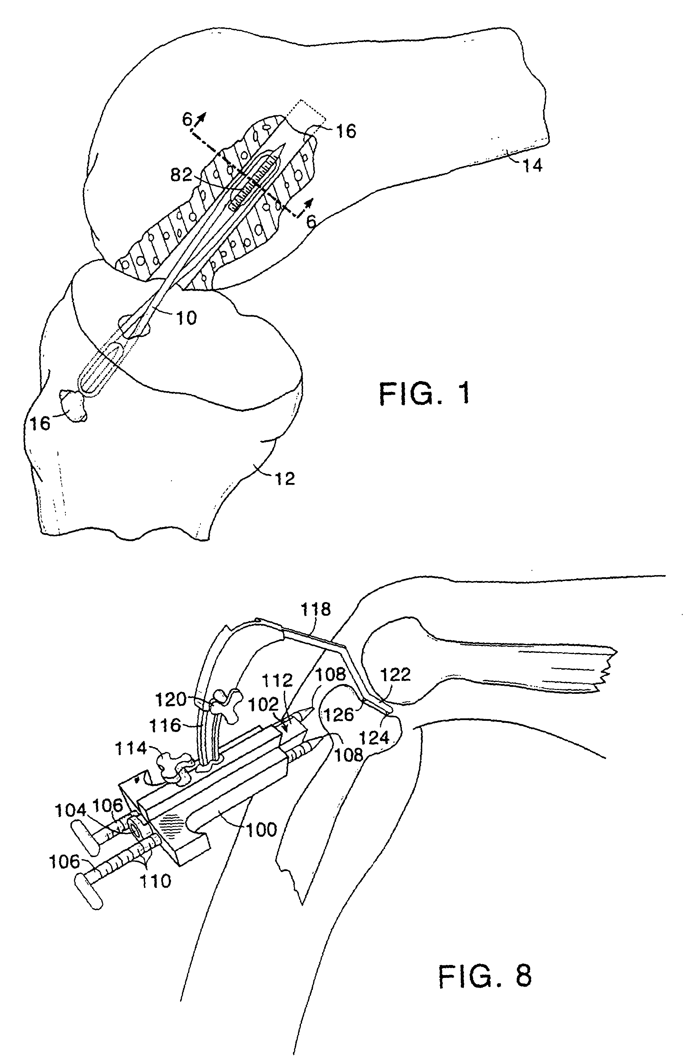

Referring now to the drawings, a reconstructed ligament **10** for a knee joint is shown in Fig. 1. The cruciate ligament reconstruction surgical operation can be conducted as an open surgery, or preferably, through arthroscopic surgery. The arthroscopic surgical method presently preferred for carrying out the present invention shall now be described.

Arthroscopic diagnostic procedures are first conducted without tourniquet control in order to allow sufficient time for the ACL reconstruction procedure. Conventional anteromedial and distal lateral portals are drilled to give access to the knee joint for these procedures. The procedures may include meniscotomy, meniscal repair, removal of loose bodies, debridement of anterior cruciate ligament tear, etc. Notchplasty may be commenced under tourniquet control. The boundary of the notchplasty should be sufficiently wide (about 2 cm.) and sufficiently posterior to include the posterior lateral femoral cortex in order to ensure accurate placement and subsequent isometry.

In order to proceed with anterior cruciate ligament reconstruction, a vertical incision is made medial to the tibial tubercle approximately 2.5 cm. in length. The skin incision may be undermined in such a fashion as to provide sufficient mobility for retraction, while harvesting the tibial and femoral bone cores. A carefully placed anteromedial tibial incision may begin approximately 1 cm. medial to the tibial tubercle and 2 cm. distal to the joint line. Conventional surgical procedures are used to excise a semitendinosus tendon, and, if desired, the accompanying gracilis. While the use of the semitendinosus tendon and gracilis is one embodiment of the invention, alternative ligament replacement materials may be substituted for use in the composite graft of the invention.

The two major bones that meet at the knee joint are the tibia **12** and the femur **14.** A bone tunnel **16** is drilled through each of these two bones. The tunnels **16** may be drilled with a regular drill that crushes and removes the bone within the tunnel. However, it is preferable to use a coring reamer to drill the bone tunnels. The reamer drills out a core of bone through each of the bone tunnels. A tibial guide **30** properly orients and guides a coring reamer for making the bone tunnels without a guide wire.

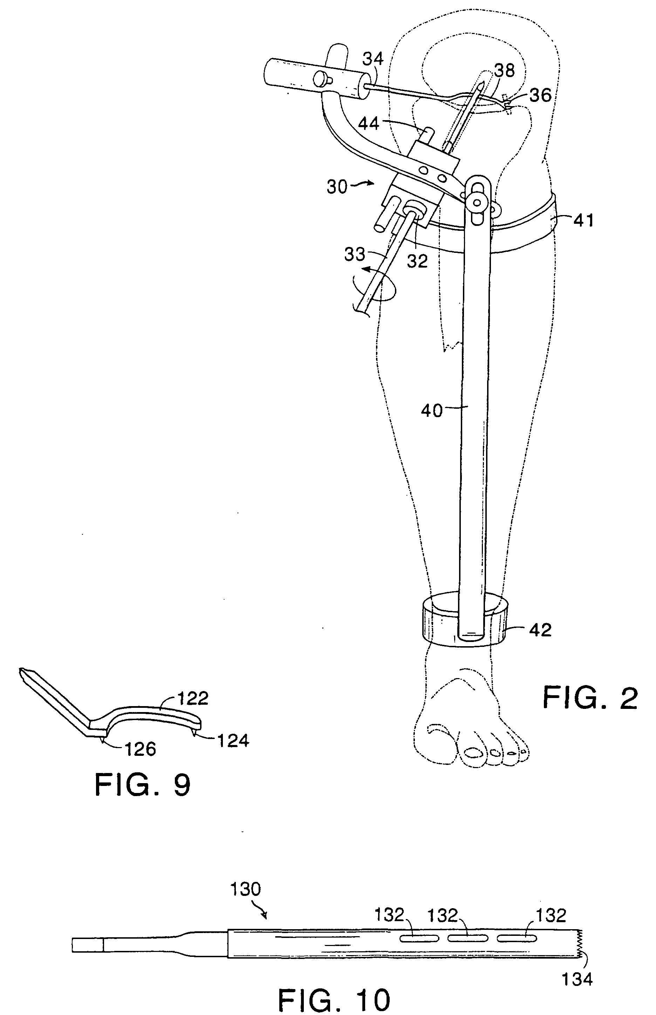

Referring now to FIG. 2, the tibial guide **30** is shown. A pipe **32** is oriented at approximately 55° to horizontal. The pipe **32** provides a cylindrical tunnel that serves to guide a coring reamer **33** or other drill inserted therethrough. With the patient's leg held fixed at approximately 110° to 120°, the guide can be used for drilling both the tibial tunnel and then the femoral tunnel. Therefore, a portal for the drill is not required behind the femur and a closed tunnel can be drilled. Both tunnels are drilled through the tibia from the anteromedial tibial incision.

A positioning arm **34** is attached to the pipe **32.** The positioning arm **34** has a fork **36** at its far end. The fork **36** has two rounded prongs. The fork **36** is attached to an arcuate portion **38** of the arm **34.** The arcuate portion **38** allows for maneuverability of the arm **34** within the knee area upon insertion through the anteromedial portal. Meanwhile, the arthroscope is inserted into the knee joint through the distal lateral portal. The fork **36** needs to be placed against the leading edge of the posterior cruciate ligament. The positioning arm **34** is shaped and oriented with respect to the pipe **32** so that the hole drilled by a reamer or drill through the pipe **32** is directed through the tibia to a point approximately **7** millimeters from the leading edge of the posterior cruciate ligament. The center of the tibial tunnel is further defined by the tangent to the center of the inner circumference of the anterior one-third of the lateral meniscus.

An adjustable rod **40** is attached to the pipe **32** at one end. A calf strap **41** secures the guide to the patient's leg. The guide has an ankle strap **42** proximate the opposite end of the rod **40.** The rod **40** can be adjusted in length to accommodate different leg sizes. The calf strap 41 and ankle strap **42** provide anchors for achieving and maintaining proper orientation of the pipe **32**. The straps are affixed with the fork **36** oriented properly around the PCL attachment on the tibia.

Another anchor to securely orient the pipe **32** is provided by a K-wire **44.** The K-wire **44** is shot through the skin of the patient's leg and into the tibia. The K-wire **44** may be positioned on the guide **30** closely adjacent the pipe **32** so that the hole formed in the tibia by the K-wire is adjacent and parallel to the hole to be drilled through the pipe **32.** The anchoring provided by the cup **36,** the ankle strap **42** and the K-wire **44** stably and correctly position the pipe **32** for guiding a coring reamer or a drill. The tunnels may thus be cored without a guide pin in the core. The tibial tunnel is reamed first and the core removed. The knee is flexed or extended a variable amount in order to properly position the femoral tunnel. A longer coring reamer is then directed through the tibial tunnel for drilling in and through the femur. The bone core from the femur is removed. Standard debarring and debridement procedures are followed.

An embodiment of a suitable tibial guide is shown in FIG. 8. Rigid stabilization of the guide against the knee is accomplished without the need for an ankle strap. A base **100** has a longitudinal Open passage for receiving a guide tube **102.** The guide tube **102** has a cylindrical tunnel **104** longitudinally therethrough. Guide tubes may be made with different diameter cylindrical tunnels to accommodate reamers or drills of corresponding diameter. The cylindrical tunnel **104** must be tight enough around the reamer to accurately guide it but wide enough to permit the reamer to rotate therein. On either side of the cylindrical tunnel, anchoring pins **106** or outriggers may be inserted. The two anchoring pins **106** provide two points of stability for the base against the tibia. The anchoring pins **106** have pointed ends **108** that can pierce skin. Therefore, it is not necessary to insert the pins through a incision, and they can be used to poke their own holes. Th anchoring pins **106** securely engage the anterior margin of the tibia. The anchoring pins are notched along their length on one side. Inside the base **100,** the notches **110** engage internal ridges to act like a ratchet. As the anchoring pins **106** are extended out from the base the notches click against the ridges. The anchoring pins **106** are prevented from retracting by the notches and ridges. To remove an anchoring pin from a patient, the handle on the proximal end of the anchoring pin is turned 180° to disengage the notches from the ridges. Then the anchoring pin can move freely longitudinally in either direction. The handle of the anchoring pin may advantageously be arranged as a flag which points horizontally outward from the base when the notches are engaged and points inward toward the other anchoring pin when the notches are disengaged. The adjustability of the anchoring pins accommodates the variation in leg size encountered from patient to patient.

Once the anchoring pins **106** have been adjusted, the guide tube **102** can also be reciprocally adjusted. The top outer surface **112** of the guide tube preferably has a flat portion for engagement with a set screw. A knob **114** on top of the base can be turned to tighten or release the screw from the guide tube. With the screw released, the guide tube **102** is pushed up against the tibia to thereby provide three points of engagement near the entrance of the tunnel to be drilled.

A curved track **116** is securely attached vertically to the base **100.** A positioning arm **118** is adjustably mountable on the curved track. A screw handle **120** on the positioning arm is used to tighten the arm **118** against the vertical track **116.** The positioning arm **118** can be slid along the track **116** to assume a range of positions. The range alters the angle made between an axis of the cylindrical tunnel of the guide tube and a seating portion **122** at the distal end of the positioning arm within a range of between about 45-50° and 80°. By providing a sufficiently large minimum angle of between 45 and 50°, the tibial guide ensures that the tibial tunnel is not drilled at too shallow an angle.

The seating portion **122** of the positioning arm at the distal end of the arm is provided with two anchoring spikes **124** and **126**. The spikes project from the end of the positioning arm for insertion into the top of the tibia. The spikes precisely define the exit end of the tunnel to be drilled through the tibia, and thus permit the surgeon to know on inspection and control the tunnel's location. Therefore, it is desirable to place the spikes so that the anatomic center of the anterior cruciate ligament is located midway therebetween. The spikes are sharp so that they may dig into the tibia. A first spike **124** extends from near the end of the positioning arm. The seating portion **122** of the positioning arm is curved between the first spike **124** and a second spike **126.** The curve defines and identifies an open region to permit clearance for a coring reamer used in operation to drill a hole through the bone. Thus, the positioning arm does not interfere with the drilling process. Moreover, the seating portion **122** partially encircles the tunnel to be drilled and the spikes are inserted on opposite sides of the tunnel to be drilled. The two spikes on the positioning arm and the two anchoring pins through the base provide four points of stabilization which make for a completely rigid attachment between the tibial guide and the tibia. The attachment is advantageously rigid in three dimensions.

With the tibial guide rigidly attached to the tibia, it is a relatively simple matter to insert the coring reamer through the cylindrical tunnel and drill an accurate tunnel. Advantageously, the bone core is not exposed to the damage ordinarily accompanying the use of a guide wire. However, without the hole through the bone caused by a guide wire, the bone core plugs up the coring reamer with almost fluid-tight engagement. Pulling the bone core out from a conventional coring reamer would be difficult because they are generally solid cylinders. Air or other fluid cannot get in behind the bone core so that pulling on the bore core tends to create a vacuum behind the bone core. The suction of the vacuum pulls the bone core into the coring reamer making it difficult to remove. A coring reamer **130** may be used that is slotted to permit air or fluid in behind the bone core as shown in FIG. 10. The slots or openings 132 are a greater distance from the cutting edge 134 of the reamer than the length of a bone core to be drilled out. A rod can be inserted through a slot or opening behind the bone core to easily push the bone core out from the reamer.

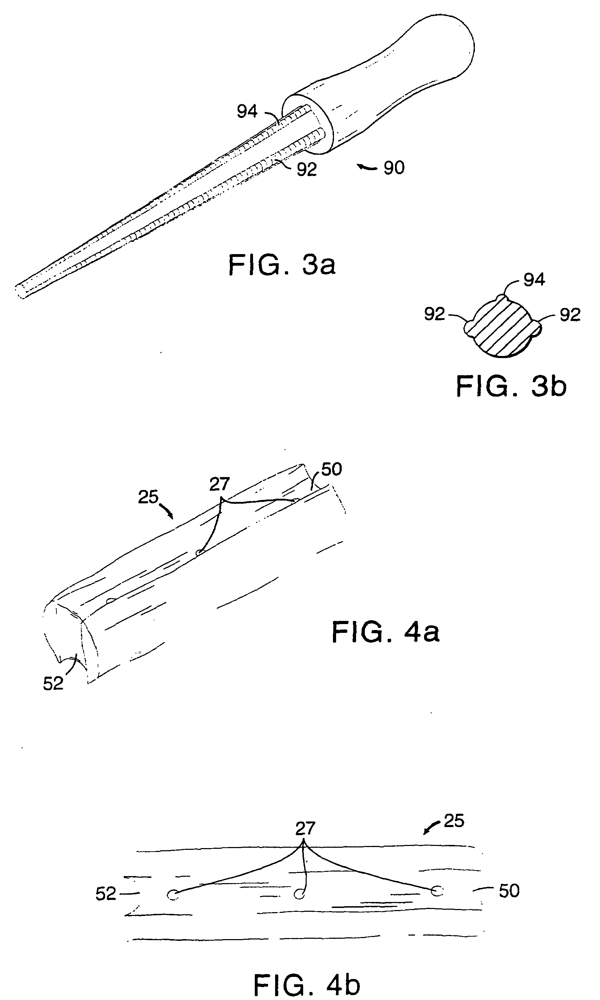

Donor bone, namely allograft bone, can be used to make the bone plugs. Referring now to FIGS. 4a and 4b, two longitudinal substantially parallel grooves **50** are drilled on opposite sides of each bone plug. The grooves provide a recess in which the semitendinosus tendon **20** and gracilis **21** can be seated. A notch **52** may also be drilled, if desired, across one end of the bone plug so that the tendon can be wrapped alongside and around the end of the bone plug, without protruding excessively from the plug. The notch **52** is not required because the bone tunnel is open at each end providing no restriction on the tendon projecting above the end of the graft. It is also advantageous to provide suture holes **27** through the bone plug for attaching the tendon to the plug. The suture holes **27** are drilled into the grooves radially through the bone plug and from one of the substantially parallel grooves **50** to the other. In a presently preferred embodiment, three such suture holes are drilled through the bone plug.

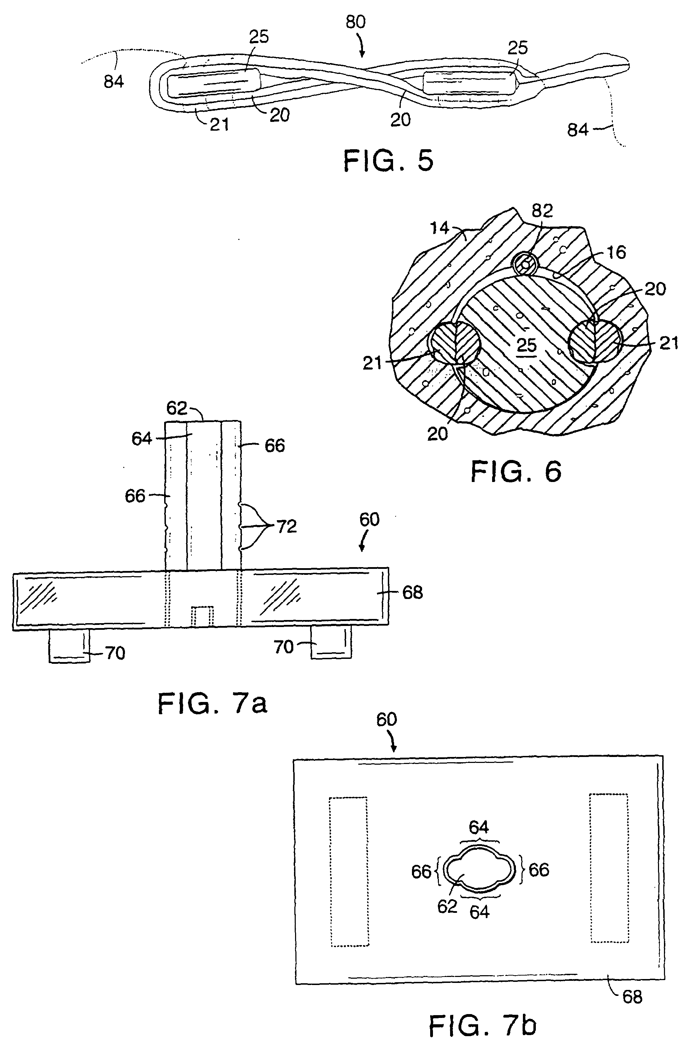

In order to easily and efficiently form a bone core into the desired bone plug for a composite graft, a bone block drill guide **60** as shown in Figs. 7a and 7b may be used. The drill guide **60** features a central substantially cylindrical column **62.** The central column **62** includes a pair of opposing curved walls **64** having a center of curvature substantially coincident with the center axis through the column **62.** The curved walls **64** are shaped so as to hold a bone core parallel with the axis of the column and substantially centered within the column. A second pair of opposing curved walls are arranged at 180° to each other with respect to the central column formed by the curved walls **64**. This second pair of walls are the drill guide walls **66**. The drill guide walls **66** form two parallel columns on opposite sides of the central column. The drill guide walls **66** have a shorter radius of curvature than the first pair of opposing curved walls **64**. In accordance with a presently preferred embodiment, the inner diameter of the drill guide walls **66** is 6 mm whereas the inner diameter of the first pair of opposing walls **64** is 11 mm. The central column **62** is mounted over a base **68.** A bone core standing in the central column **62** rests on the base **68.** The base **68** is provided with holes therethrough in alignment with the open circular cylinder formed within the drill guide walls **66.** The base **68** may also include legs **70** for supporting the drill guide over a table. For drilling suture holes through the bone block, holes **72** are arranged horizontally through the drill guide walls **66**. Three holes **72** are preferably aligned in a line.

The substantially parallel grooves **50** are drilled by inserting the bone core or allograft into the center chamber of the column **62** formed by the opposing curved walls **64**. A drill is directed in the column **62** along each of the drill guide walls **66** in succession. Thus, parallel grooves **50** are formed on opposite sides of the bone core. The drill may be equipped with a stop to prevent the drill from being directed too far down through the column where it may contact the table beneath. A drill bit inserted through the holes **72** can be easily directed through the center of a groove drilled along the bone core. The suture holes drilled through guide holes **72** preferably extend from one groove to the opposite groove in the bone block.

The semitendinosus tendon **20** and/or gracilis **21** is extended between both of the bone plugs **25.** The tendons are seated inside the two substantially parallel grooves **50** and about an end of each bone plug. The tendons are preferably sutured to themselves to form a double loop as shown in FIG. 5. Sutures are also used through the suture holes to attach the tendon to each of the bone plugs. The tendon strands may be straight or twisted between the bone plugs. Twisting will shorten the length of the graft. A ligament replacement of an embodiment of the invention may include both the semitendinosus tendon and the gracilis. As such four strands will connect the two bone plugs. Other embodiments of the invention may use one or the other of the semitendinosus tendon and gracilis. Still further embodiments of the invention may substitute or combine man made or artificial fibers or human tissue for the tendons for use as the ligament replacement.

In affixing the composite graft **80** within a bone tunnel, contact between a screw **82** and the tendon should be avoided so as not to cut or tear the tendon. To better insure that the screw is out of contact with the tendon, an interference screw should be driven along the bone portion of the graft between the graft and the bone tunnel wall. A trefoil rasp **90** is recommended for use prior to fixation of the graft. As shown in Figs. 3a and 3b, the trefoil rasp **90** has three longitudinal lobes for use in cutting three channels into each of the bone tunnels. Reciprocating movement of the trefoil rasp **90** in and out of the bone tunnels **16** serves to file away the tunnel walls to form the desired channels. Two of the longitudinal lobes **92** are 180° apart on the rasp. These longitudinal lobes **92** are used to form channels for accommodating the semitendinosus tendons **20** and gracilis **21** seated in the parallel grooves of the bone graft. When the gracilis **21** is attached along and on top of the semitendinosus tendon 20, the channels are required to provide room for the graft to fit within the tunnel.

The third longitudinal lobe **94** is located parallel to and equidistant from the two opposed lobes. Looking at the end of the rasp as in Fig. 3b, the third lobe **94** is preferably 90° to each of the other two lobes. In one embodiment, the third lobe **94** projects 2 mm. from the rasp shaft whereas the other two lobes **92** each project 3 mm. from the shaft. The third lobe **94** advantageously files away a channel along which an interference screw is driven as shown in FIG. 6. The channel helps to maintain the screw straight adjacent the bone portion of the graft. Advantageously, the tibial guide **30** and the trefoil rasp **90** can complement one another in forming the channel for the screw. The hole formed by the K-wire **44** of the tibial guide may be used as the screw channel. To achieve this result, the trefoil rasp should be aligned in the tibial tunnel with its third lobe **94** overlapping into the K-wire hole.

After the channels have been filed in the bone tunnels, the sutures **84** hanging from one end of the composite graft are attached to a needle, a passer or other conventional graft placement tool. The passer is inserted through tibial and femoral bone tunnels and out through the skin on the posterior side of the knee. The passer is removed leaving the suture hanging from the posterior end of the graft and a suture at the other end of the graft hanging out through the tibial incision. The sutures may be pulled on to properly tension and locate the graft within the bone tunnels. Alternatively, the graft may be positioned within the bone tunnels using a pushing device instead of a suture pulling the graft into position.

Fixation of the graft is preferably accomplished with a headless cannulated interference screw. The cannulated interference screw can be carried by a guide wire extending from the tip of an angled driver. The guide wire is preferably a springy wire made of a material such as Nitinol^{tm}. The wire extends about 2 centimeters past the end of a screw carried by the driver. For securing the interference screw in the femoral tunnel, the angled or flexible driver and screw are preferably inserted through the anteromedial portal. An angled driver and use thereof is described in co-pending United States Patent Application Serial No. 07/956,733 filed October 2, 1992. A flexible slide may be used to provide a track to follow from the anteromedial portal to the channel in the femoral tunnel for the interference screw. The insertion of a flexible slide simplifies the guidance of the interference screw into the channel of the femoral tunnel. Once the screw is properly positioned in the tunnel, the driver can initiate screwing and the slide can be removed. The oppositely located channels in the femoral tunnel hold the semitendinosus tendon in position away from the interference screw as it is screwed between the bone portion of the graft and the channel of the bone tunnel. Upon fixation of the interference screw in the femoral tunnel, the angled driver is removed.

The proper tension is then applied to the graft by pulling on the suture hanging out from the tibial incision. A driver and a headless cannulated interference screw are then inserted through the tibial incision for driving the screw along the channel formed in the tibial tunnel. The sutures are cut and the incisions are closed. The reconstructed knee upon fixation of the graft appears as in Fig. 1.

While this operation has been discussed in terms of using autogenous bone cores, alternative sources of bone plugs are substituted in the present invention. Allografts, in which donor bone is freeze-dried or fresh frozen for preservation, are one alternative. The freeze drying process kills cells in the bone and may reduce the risk of transmission of infection. Another alternative bone plug is the use of synthetic graft material. With any of these alternatives, the bone plugs may be shaped to appear as described above for the autogenous graft. With the allograft and the synthetic graft, the coring reamer is no longer required and an ordinary drill may be used instead for drilling the bone tunnels.

The surgical technique of the present invention advantageously makes use of the fact that the semitendinosus and gracilis has less morbidity associated with harvesting than does the patellar tendon. It is further advantageous to use a coring reamer and a bone block drill guide to remove the bone cores from the bone tunnels in the tibia and femur and shape them to accommodate the semitendinosus tendon. The trefoil rasp provides the still further advantage of maintaining alignment of the graft and interference screws in the bone tunnel so that the screw is directed adjacent only the bone portion of the graft.

Of course, it should be understood that various changes and modifications to the preferred embodiments described above will be apparent to those skilled in the art. For example, the bone blocks in the composite graft may be allogenic or synthetic. The tendon or other ligament replacement used on the graft may be one or more strands sutured to both of the bone blocks along the grooves. Moreover, alternative equipment may be used for drilling the grooves and the bone plugs.

## Claims

1. A composite graft (80) comprising first and second bone plugs and a ligament replacement **characterized in that** it comprises: first and second allogenic or synthetic bone plugs (25), each having two substantially parallel longitudinal grooves (50) formed along opposite sides of said each bone plug and lacking any other longitudinal holes cut therein; and
a ligament replacement formed in a loop (20, 21), wherein said ligament replacement is either an allograft or a synthetic graft extending around said first and second bone plugs (25) along said grooves (50) and being sutured to each of said first and second bone plugs (25).

2. The composite of Claim 1, wherein said first and second bone plugs (25) further comprise at least one suture hole (27) drilled radially through each of said bone plugs (25) from one of the longitudinal grooves (50) through to the other substantially parallel longitudinal groove (50).

3. The composite graft of Claim 1 or 2, wherein said ligament replacement comprises a semitendinosus tendon (20).

4. The composite graft of Claim 3, wherein said ligament replacement further comprises a gracilis (21).

5. The composite graft according to Claim 1 further comprising: a screw (82) forming an interference fixation between the first bone plug (25) and a tunnel in a first bone; and
a screw (82) forming an interference fixation between the second bone plug (25) and a tunnel in a second bone.

6. The composite graft of Claim 5, wherein said first and second bone plugs (25) each have at least one suture hole (27) extending radially through said bone plug (25) from one of the longitudinal grooves (50) through to the other substantially parallel groove and wherein a suture extends through the suture hole in each of said bone plugs and said first and second ligament replacement strands (20, 21) to attach said first and second ligament replacement strands to said first and second bone plugs.

7. The composite graft of Claim 6, wherein said first and second ligament replacement strands (20, 21) are formed by a semitendinosus tendon (20) looped around said first and second bone plugs.

8. The graft of Claim 1 or Claim 7, wherein said first and second bone plugs further include a notch (52) in one end for wrapping said semitendinosus tendon (20) over from one longitudinal groove (50) to the other substantially parallel longitudinal groove.

9. The composite graft according to Claim 1, wherein the ligament replacement used on the graft may be one or more strands.

## Patentansprüche

1. Verbundimplantat (80) mit ersten und zweiten Knochenzapfen und einem Bandersatz, **dadurch gekennzeichnet, dass** es umfasst: erste und zweite allogene oder synthetische Knochenzapfen (25), von denen jeder zwei im wesentlichen parallele Longitudinalrillen (50) aufweist, die entlang gegenüberliegenden Seiten jedes der Knochenzapfen ausgebildet sind und nicht irgendwelche weiteren, darin eingeschnittenen longitudinalen Ausnehmungen aufweisen, und
einen in einer Schleife (20,21) ausgebildeten Bandersatz, wobei der Bandersatz entweder ein Allo-Implantat oder ein synthetisches Implantat ist, das sich um die ersten und zweiten Knochenzapfen (25) entlang der Rillen (50) erstreckt und an jedem der ersten und zweiten Knochenzapfen (25) angenäht ist.

2. Verbundimplantat nach Anspruch 1, wobei die ersten und zweiten Knochenzapfen (25) ferner mindestens ein Nahtloch (27) aufweisen, das radial durch jeden der Knochenzapfen (25) von einer der longitudinalen Rillen (50) durch die andere, im wesentlichen parallele longitudinale Rille (50) gebohrt ist.

3. Verbundimplantat nach Anspruch 1 oder 2, wobei der Bandersatz eine halbsehnige Sehne (semitendinosus tendon) (20) umfasst.

4. Verbundimplantat nach Anspruch 3, wobei der Bandersatz ferner einen Gracilis (21) umfasst.

5. Verbundimplantat nach Anspruch 1, ferner mit: einer Schraube (82), die eine Interferenzbefestigung zwischen dem ersten Knochenzapfen (25) und einem Tunnel in einem ersten Knochen bildet, und
einer Schraube (82), die eine Interferenzbefestigung zwischen dem zweiten Knochenzapfen (25) und einem Tunnel in einem zweiten Knochen bildet.

6. Verbundimplantat nach Anspruch 5, wobei die ersten und zweiten Knochenzapfen (25) jeweils mindestens ein Nahtloch (27) aufweisen, das sich radial durch den Knochenzapfen (25) von einer der Longitudinalrillen (50) bis zu der anderen, im wesentlichen parallelen Rille erstreckt, und wobei sich eine Naht durch das Nahtloch in jedem der Knochenzapfen und die ersten und zweiten Bandersatzstränge (20,21) erstreckt, um die ersten und zweiten Bandersatzstränge an den ersten und zweiten Knochenzapfen zu befestigen.

7. Verbundimplantat nach Anspruch 6, wobei die ersten und zweiten Bandersatzstränge (20,21) durch eine halbsehnige Sehne (semitendinosus tendon) (20) gebildet sind, die um die ersten und zweiten Knochenzapfen schleifenartig herumgeführt ist.

8. Implantat nach Anspruch 1 oder Anspruch 7, wobei die ersten und zweiten Knochenzapfen ferner eine Einkerbung (52) in einem Ende zum Herumwickeln der halbsehnigen Sehne (20) von einer longitudinalen Rille (50) zu der anderen, im wesentlichen parallelen longitudinalen Rille hin aufweist.

9. Verbundimplantat nach Anspruch 1, wobei der am Implantat verwendete Bandersatz einen oder mehrere Stränge aufweisen kann.

## Revendications

1. Greffe composite (80) comprenant des premier et second bouchons osseux et une prothèse de ligament **caractérisée en ce qu'**elle comprend : des premier et second bouchons osseux allogènes ou synthétiques (25), ayant chacun deux rainures longitudinales sensiblement parallèles (50) formées le long de côtés opposés de chacun desdits bouchons osseux et ne comprenant pas de trous longitudinaux découpés dans ceux-ci ; et
une prothèse de ligament formée en une boucle (20, 21), dans laquelle ladite prothèse de ligament est une allogreffe ou une greffe synthétique s'étendant autour desdits premier et second bouchons osseux (25) le long desdites rainures (50) et étant suturée à chacun desdits premier et second bouchons osseux (25).

2. Composite selon la revendication 1, dans lequel desdits premier et second bouchons osseux (25) comprennent en outre au moins un trou de suture (27) percé radialement à travers chacun desdites bouchons osseux (25) de l'une des rainures longitudinales (50) à l'autre rainure longitudinale sensiblement parallèle (50).

3. Greffe composite selon la revendication 1 ou 2, dans laquelle ladite prothèse de ligament comprend un tendon demi-tendineux (20).

4. Greffe composite selon la revendication 3, dans laquelle ladite prothèse de ligament comprend en outre un muscle droit interne (21).

5. Greffe composite selon la revendication 1 comprenant en outre une vis (82) formant une fixation d'interférence entre le premier bouchon osseux (25) et un tunnel dans un premier os ; et
une vis (82) formant une fixation d'interférence entre le second bouchon osseux (25) et un tunnel dans un second os.

6. Greffe composite selon la revendication 5, dans laquelle lesdits premier et second bouchons osseux (25) ont chacun au moins un trou de suture (27) s'étendant radialement à travers ledit bouchon osseux (25) de l'une des rainures longitudinales (50) à l'autre rainure sensiblement parallèle et dans laquelle une suture s'étend à travers le trou de suture dans chacun desdits bouchons osseux et lesdits premier et second brins de prothèse de ligament (20, 21) pour attacher lesdits premier et second brins de prothèse de ligament auxdits premier et second bouchons osseux.

7. Greffe composite selon la revendication 6, dans laquelle lesdits premier et second brins de prothèse de ligament (20, 21) sont formés par un tendon demi-tendineux (20) en boucle autour desdits premier et second bouchons osseux.

8. Greffe selon la revendication 1 ou la revendication 7, dans laquelle lesdits premier et second bouchons osseux comprennent une encoche (52) à une extrémité pour entourer ledit tendon demi-tendineux (20) d'une rainure longitudinale (50) à l'autre rainure longitudinale sensiblement parallèle.

9. Greffe composite selon la revendication 1, dans laquelle la prothèse de ligament utilisée sur la greffe peut être un ou plusieurs brins.
